(19) Europäisches Patentamt
European Patent Office
Office européen des brevets

(11) Veröffentlichungsnummer: **0 131 801**
**A1**

(12) **EUROPÄISCHE PATENTANMELDUNG**

(21) Anmeldenummer: **84107382.8**

(22) Anmeldetag: **27.06.84**

(51) Int. Cl.⁴: **C 07 D 233/32**

(30) Priorität: **09.07.83 DE 3324903**

(43) Veröffentlichungstag der Anmeldung:
**23.01.85 Patentblatt 85/4**

(84) Benannte Vertragsstaaten:
**CH DE FR GB IT LI**

(71) Anmelder: **BAYER AG**
**Konzernverwaltung RP Patentabteilung**
**D-5090 Leverkusen 1 Bayerwerk(DE)**

(72) Erfinder: **Buysch, Hans-Josef, Dr.**
**Brandenburgerstrasse 28**
**D-4150 Krefeld(DE)**

(72) Erfinder: **Klipper, Reinhold, Dr.**
**Bismarckstrasse 13**
**D-5000 Koeln 50(DE)**

(72) Erfinder: **Lange, Peter Michael, Dr.**
**Walter-Flex-Strasse 9**
**D-5090 Leverkusen 1(DE)**

(72) Erfinder: **Mues, Peter, Dr.**
**Breslauer Strasse 31**
**D-4150 Krefeld 11(DE)**

(54) **Verfahren zur Herstellung substituierter Ethylenharnstoffe sowie neue N-Vinylethylenharnstoffe.**

(57) Substituierte Ethylenharnstoffe werden hergestellt durch Umsetzung entsprechender ß-Hydroxyethylharnstoffe mit Kohlensäurealkylestern in Gegenwart von Katalysatoren bei erhöhter Temperatur.

Substituierte Ethylenharnstoffe dienen u,a, als Polymerisations- und Copolymerisationskomponenten zur Herstellung von Kunststoffen, Überzügen, Lacken und Ionenaustauschern.

EP 0 131 801 A1

Croydon Printing Company Ltd

BAYER AKTIENGESELLSCHAFT 5090 Leverkusen, Bayerwerk

Konzernverwaltung RP
Patentabteilung Bg/bc/c 08. 07. 83

# Verfahren zur Herstellung substituierter Ethylenharnstoffe sowie neue N-Vinylethylenharnstoffe

Die Erfindung betrifft ein Verfahren zur Herstellung substituierter Ethylenharnstoffe sowie neue N-Vinylethylenharnstoffe.

Die Herstellung von disubstituierten Ethylenharnstoffen durch Anlagerung von Epoxiden an Carbodiimiden ist z.B. aus Chem. Ber. 94, 3287 (1961) bekannt. Nachteilig bei diesem Verfahren sind die erforderlichen hohen Reaktionstemperaturen (200°C), langen Reaktionszeiten (3 bis 10 Stunden) und die teuren Einsatzprodukte. Weiterhin ist bekannt, substituierte Ethylenharnstoffe herzustellen durch Umsetzung von substituierten Ethylendiaminen mit Phosgen oder Kohlensäurederivaten zu Carbaminsäureestern und deren thermische Cyclisierung (J. Org. Chem. 26, 4051 (1961)). Dieses Verfahren besitzt den Nachteil, daß die einzusetzenden Ethylendiamine häufig schlecht zugänglich und daher recht teuer sind. Gemäß der DE-OS 2 230 076 und der DE-OS 2 035 364 können substituierte Ethylenharnstoffe hergestellt werden durch Cyclisierung von N-ß-Chlorethylharnstoffen unter Abspaltung von Halogenwasserstoff. Nachteilig an diesem Verfahren ist die Verwendung von stark korrosivem Thionylchlorid zur Herstellung der N-ß-Chlorethylharnstoffe aus den entsprechenden N-ß-Hydroxyethylharnstoffen und bei der Cyclisierung die Bildung von Chlorwasserstoff. bzw. Natriumchlorid. Ein ähnliches Cyclisierungsverfahren zur Herstellung von substituierten Ethylenharnstoffen ist aus Helv. Chim. Acta 49, 2400 (1966) und aus Bull. Chem. Soc. Japan 39, 708 (1966) bekannt. Bei den dort geschilderten Cyclisierungsverfahren stellt die Sulfat- bzw. die Phosphitgruppe die austretende Gruppe dar. Nachteilig bei diesem Verfahren sind die unbefriedigenden Ausbeuten, die sich u.a. durch die Einführung der Austrittsgruppe ergeben, und die Beseitigung der bei der Cyclisierung entstehenden Abfallprodukte. Bekannt ist ferner die Herstellung von N-Vinylethylenharnstoff, N,N'-Divinylethylenharnstoff und von N-Ethyl-N'-vinylethylenharnstoff (vgl. US 2 541 152 und J. Macromol. Sci. Chem. A9, 1085 (1975)). Den Verfahren zur Herstellung von Vinylethylenharnstoffen ist gemein, daß sie von vorgegebenem Ethylenharnstoff ausgehen, wobei entweder das Kaliumsalz des Harnstoffs mit Acetylen unter Druck umgesetzt wird oder die Vinylverbindung in einer vielstufigen Synthese über einen Hofmann-Abbau gewonnen wird. Nachteilig bei diesen Verfahren sind zum einen die umfangreichen Sicherheitsmaßnahmen und die technisch aufwendigen Apparaturen, die beim Arbeiten mit Acetylen unter Druck erforderlich sind, zum anderen ist

die Synthese über den Hofmann-Abbau umständlich und mit dem Einsatz von teuren Ausgangsprodukten verbunden.

Es wurde nun ein Verfahren zur Herstellung von substituierten Ethylenharnstoffen der Formel (I)

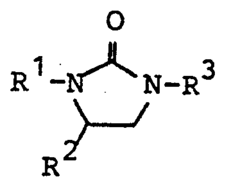

(I) ,

worin

$R^1$ für einen gegebenenfalls durch Chlor, Brom, eine Nitro-, Trifluormethyl-, Alkoxygruppe mit 1 bis 4 Kohlenstoffatomen oder eine Carbalkoxygruppe mit 1 bis 4 Kohlenstoffatomen ein- oder mehrfach substituierten Phenylrest steht,

$R^2$ Wasserstoff oder Methyl bedeutet und

$R^3$ für einen gegebenenfalls durch Chlor, Brom, eine Nitro-, Trifluormethyl-, Alkoxygruppe mit 1 bis 4 Kohlenstoffatomen oder eine Carbalkoxygruppe mit 1 bis 4 Kohlenstoffatomen ein- oder mehrfach substituierten Phenylrest, einen Alkylrest mit 1 bis 4 Kohlenstoffatomen, für $-CH_2CHR^2O\overset{O}{\overset{\|}{C}}OR^4$ mit $R^4$ für $C_1$-$C_4$-Alkyl oder für $-CH=CHR^2$, mit der für $R^2$ genannten Bedeutung steht,

gefunden, daß dadurch gekennzeichnet ist, daß man ß-Hydroxyethylharnstoffe der Formel (II)

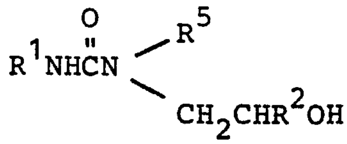

(II) ,

worin
$R^1$ und $R^2$ die oben angegebene Bedeutung haben und
$R^5$ für einen gegebenenfalls durch Chlor, Brom, eine Nitro-, Trifluormethyl-, Alkoxygruppe mit 1 bis 4 Kohlenstoffatomen oder durch eine Carbalkoxygruppe mit 1 bis 4 Kohlenstoffatomen ein- oder mehrfach substituierten Phenylrest, einen Alkylrest mit 1 bis 4 Kohlenstoffatomen oder für $-CH_2CHR^2OH$ mit der für $R^2$ genannten Bedeutung steht,

mit Kohlensäurealkylestern der Formel (III)

$$R^6O\overset{O}{\overset{\|}{C}}OR^7 \qquad (III),$$

worin
$R^6$ und $R^7$ gleich oder verschieden sind und für einen Alkylrest mit 1 bis 4 Kohlenstoffatomen stehen,

in Gegenwart von Katalysatoren bei erhöhter Temperatur umsetzt.

Als Alkylreste mit 1 bis 4, bevorzugt 1 bis 2, Kohlenstoffatomen der Formeln (I) bis (III) seien genannt: der Methyl-, der Ethyl-, der n-Propyl-, der Isopropyl-, der n-Butyl-, der Isobutyl- und tert.-Butyl-Rest, bevorzugt der Methyl- und der Ethylrest.

Als Alkoxygruppen mit 1 bis 4, bevorzugt 1 bis 2 Kohlenstoffatomen der Formeln (I) und (II) seien genannt: der Methoxy-, der Ethoxy-, der n-Propyloxy-, der Isopropyloxy-, der n-Butyloxy-, der Isobutyloxy- und der

tert.-Butyloxy-Rest, bevorzugt der Methoxy und Ethoxyrest und als Carbalkoxygruppen mit 1 bis 4, bevorzugt 1 bis 2 Kohlenstoffatomen der Formeln (I) und (II) seien genannt: Methoxycarbonyl, Ethoxycarbonyl, n-Propyloxycarbonyl, Isopropyloxycarbonyl, n-Butyloxycarbonyl, Isobutyloxycarbonyl und tert.-Butyloxycarbonyl, bevorzugt Methoxycarbonyl und Ethoxycarbonyl.

Zum Beispiel können als Verbindungen der Formel (II) in das erfindungsgemäße Verfahren eingesetzt werden:

N-Phenyl-N'-2-hydroxyethyl-N'-methylharnstoff, N-(3,4-Dichlorphenyl)-N'-2-hydroxyethyl-N'-methylharnstoff, N-Phenyl-N'-2-hydroxyethyl-N'-phenylharnstoff, N-Phenyl-N',N'-bis-(2-hydroxyethyl-harnstoff, N-(3,4-Dichlorphenyl)-N',N'-bis-(2-hydroxyethyl)-harnstoff, N-(4-Nitrophenyl)-N',N'-bis-(2-hydroxyethyl)-harnstoff, N-(4-Ethoxyphenyl)-N',N'-bis-(2-hydroxyethyl)-harnstoff oder N-(4-Methylphenyl)-N',N'-bis-(2-hydroxyethyl)-harnstoff.

Die o.g. ß-Hydroxyethylharnstoffe können auf einfache Weise durch Addition von Aminen der Formel $HNR^5CH_2CHR^2OH$ mit der o.g. Bedeutung für $R^2$ und $R^5$ an Isocyanate der Formel $R^1NCO$ mit der für $R^1$ genannten Bedeutung in Gegenwart eines inerten organischen Lösungsmittels, wie Methylenchlorid, Ethylenchlorid, Chlorbenzol und/oder o-Dichlorbenzol, oder direkt in Gegenwart eines zur nachfolgenden Umesterung einzusetzenden Kohlensäureesters, wie Dimethylcarbonat oder Diethylcarbonat,

erhalten werden. Die ß-Hydroxyethylharnstoffe der Formel (II) können ebenfalls erhalten werden durch eine Aminolyse entsprechender Urethane mit Aminen der Formel $HNR^5CH_2CHR^2OH$ mit der für $R^2$ und $R^5$ genannten Bedeutung. Sowohl die Addition der Amine an die Isocyanate als auch die Aminolyse von Urethanen wird unter den hierfür üblichen Reaktionsbedingungen durchgeführt (vgl. "Methoden zur Herstellung und Umwandlung von substituierten Harnstoffen, Semicarbaziden, Isoharnstoffen", Houben-Weyl, 4. Aufl., Bd. 8, S. 149 ff).

Als Isocyanate der Formel $R^1NCO$ seien z.B. genannt:

Isocyanatobenzol, 1-Chlor-3-isocyanatobenzol, 1-Chlor-4-isocyanatobenzol, 1,2-Dichlor-4-isocyanatobenzol, 1-Isocyanato-2-nitrobenzol, 1-Isocyanato-4-nitrobenzol, 1-Isocyanato-2-methylbenzol, 1-Isocyanato-4-methylbenzol, 2-Chlor-4-isocyanato-1-trifluormethylbenzol, 1-Ethoxy-4-isocyanatobenzol und 1-Carbethoxy-4-isocyanatobenzol; als Amine der Formel $HNR^5CH_2CHR^2OH$: 2-Methyl-aminoethanol, 2-Ethyl-aminoethanol, N-(2-Hydroxyethyl)-anilin, 1-Methylamino-2-propanol, Bis-(2-hydroxyethyl)-amin und Bis-(2-hydroxypropyl)-amin.

Nach dem erfindungsgemäßen Verfahren wird die Umsetzung der ß-Hydroxyethylharnstoffe der allgemeinen Formel (II) mit Kohlensäureestern der allgemeinen Formel (III) in Gegenwart von Katalysatoren bei Temperaturen im Bereich von etwa 100 bis 270°C, vorzugsweise bei 110 bis 160°C, durchgeführt.

Als Kohlensäurealkylester der Formel (III) seien solche genannt, deren Alkylreste 1 bis 4 C-Atome, bevorzugt 1 bis 2 C-Atome, enthalten, wie der Dimethyl-, Diethyl-, Di-n-propyl-, Diisopropyl-, Di-n-butyl-, Diisobutyl- und der Di-tert.-butylester der Kohlensäure, bevorzugt der Kohlensäuredimethyl- und der Kohlensäurediethylester.

Bezogen auf den eingesetzten ß-Hydroxyethylharnstoff der Formel (II) kann der Kohlensäurealkylester im Unterschuß, im Überschuß oder in äquimolaren Mengen eingesetzt werden. Bevorzugt wird ein Molverhältnis des einzusetzenden ß-Hydroxyethylharnstoffs zu einzusetzendem Kohlensäurealkylester gewählt, das etwa 1:1 bis 1:20, besonders bevorzugt 1:4 bis 1:8, beträgt.

Die Umsetzung der ß-Hydroxyethylharnstoffe mit dem Kohlensäurealkylester kann mit oder ohne ein inertes organisches Lösungsmittel durchgeführt werden. Als inerte organische Lösungsmittel kommen z.B. in Frage: Xylole, halogenierte Kohlenwasserstoffe, wie o-Dichlorbenzol, oder Ether, wie Anisol.

Als Katalysatoren, die in Mengen von etwa 0,001 bis 5 Gew.-%, bevorzugt 0,01 bis 1 Gew.-%, bezogen auf die Menge an eingesetzter Verbindung der Formel (II), eingesetzt werden, seien genannt: Die Verbindungen des Lithiums, Natriums, Kaliums, Rubidiums, Cäsiums, Magnesiums, Calciums, Zinks, Strontiums, Cadmiums, Bariums, Thalliums, Titans, Zinns und/oder des Bleis,

bevorzugt die Verbindungen der Alkali- und/oder Erdalkalimetalle. Im allgemeinen werden die Hydroxide, Alkoholate oder die alkalisch reagierenden Salze, wie die Carbonate oder die Carboxylate, der genannten Metalle eingesetzt. Bevorzugt werden in das erfindungsgemäße Verfahren eingesetzt die Carbonate, Carboxylate, die Alkoholate und/oder die Hydroxide des Natriums und/oder des Kaliums, wie Natriumhydroxid, Natriumethanolat und/oder Kaliumcarbonat.

Nach dem erfindungsgemäßen Verfahren werden die substituierten Ethylenharnstoffe der Formel (I) in bevorzugter Weise durch Destillation unter vermindertem Druck (ca. 0,01 bis 100 mbar) isoliert.

Gegenstand der Erfindung sind ebenfalls neue N-Vinylethylenharnstoffe der Formel (IV)

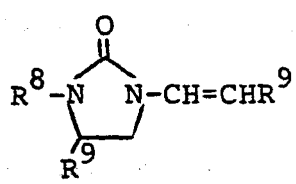

(IV) ,

worin

$R^8$ für einen gegebenenfalls durch Chlor, Brom, eine Nitro-, Trifluormethyl-, Alkoxygruppe mit 1 bis 4 Kohlenstoffatomen oder Carbalkoxygruppe mit 1 bis 4 Kohlenstoffatomen ein- oder mehrfach substituierten Phenylrest steht und

$R^9$ Wasserstoff oder Methyl bedeutet.

Als neue N-Vinylethylenharnstoffe, die nach dem erfindungsgemäßen Verfahren erhalten werden können, seien genannt: N-Phenyl-N'-vinylethylenharnstoff, N-(3,4-Dichlorphenyl)-N'-vinylethylenharnstoff, N-(4-Nitrophenyl)-N'-vinylethylenharnstoff, N-(4-Ethoxyphenyl)-N'-vinylethylenharnstoff, N-(4-Methylphenyl)-N'-vinylethylenharnstoff, bevorzugt N-Phenyl-N'-vinylethylenharnstoff und N-(3,4-Dichlorphenyl)-N'-vinylethylenharnstoff.

Die neuen N-Vinylethylenharnstoffe werden nach dem erfindungsgemäßen Verfahren erhalten, wenn man von z.B. ß-Hydroxyethylharnstoffen der Formel (II) (mit $R^5$=$-CH_2CHR^2OH$) ausgeht und diese in der beschriebenen Weise mit Kohlensäurealkylestern der Formel (III) umsetzt.

Dabei ist es nur unter bestimmten Bedingungen möglich, die zunächst gebildeten Ethylenharnstoffe der Formel (V)

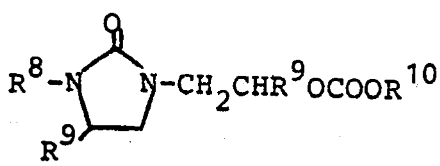

(V)

worin

$R^8$ und $R^9$ die zuvor genannte Bedeutung (Formel IV) haben und

$R^{10}$ für $C_1$-$C_4$-Alkyl steht,

zu isolieren.

Die Ethylenharnstoffe der Formel (V) sind ebenfalls neu.

In vorteilhafter Weise gelingt die Isolierung der Ethylenharnstoffe der Formel (V), indem man die Katalysatoren durch beispielsweise Extraktion mit Wasser oder mit verdünnten Säuren, wie Salzsäure, Schwefelsäure oder Essigsäure, oder durch ein Adsorptionsverfahren, wie die Adsorption an Aktivkohle oder Kieselgur, aus dem Reaktionsgemisch entfernt. Weiterhin ist es möglich, Ionenaustauscher zur Entfernung der Katalysatoren einzusetzen. Solche Ionenaustauscher sind beispielsweise säureaktivierte Bleicherden oder Austauscherharze auf der Basis sulfonierter Styrol-Divinyl-Benzol-Copolymerisate.

Aus den Ethylenharnstoffen der Formel (V) kann man die N-Vinylethylenharnstoffe der Formel (IV) leicht ohne Entfernung des im Reaktionsgemisch vorhandenen Katalysators und durch Erhitzen auf Temperaturen von etwa 160 bis 270°C, bevorzugt 180 bis 250°C, und anschließender Destillation unter vermindertem Druck bei etwa 0.01 bis 100, bevorzugt 0.01 bis 20 mbar, erhalten.

Zur weiteren Reinigung des dabei erhaltenen Produktes kann aus einem geeigneten Lösungsmittel, wie Toluol, Ethylacetat und/oder Ethanol umkristallisiert werden.

Der bei der Reaktion entstehende Alkohol, beispielsweise Methanol oder Ethanol, wird zweckmäßigerweise in einer mit Trockeneis beschickten Kühlfalle kondensiert, während das Kohlendioxid entweder in einer mit flüssigem Stickstoff gekühlten Falle oder in einer geeigneten Flüssigkeit, wie Ethanolamin oder Ethylendiamin, unter Carbaminatbildung aufgefangen wird.

Ethylenharnstoffe der Formel (IV) sind wertvolle Vorprodukte. Sie dienen u.a. als Polymerisations- und Copolymerisationskomponenten zur Herstellung von Kunststoffen, Überzügen, Lacken und Ionenaustauschern. Beispielsweise läßt sich ein Produkt mit guten ionenaustauschenden Eigenschaften nach folgenden allgemeinen Vorgehen erhalten:

In einem Reaktionsgefäß bestückt mit Thermometer, Rührer und Rückflußkühler wird bei 60°C eine wässrige Phase, bestehend aus dest. Wasser und einem darin gelösten Suspensionsstabilisator auf Cellulosebasis vorgelegt.

Unter Rühren gibt man die gelöste organische Phase hinzu, bestehend aus N-Phenyl-N'-vinyl-ethylenharnstoff, einer mehrfach ungesättigten Verbindung wie Divinylbenzol oder 1,5-Hexadien-3,4-diolcarbonat, einem Polymerisationsinitiator (z.B. Azobisisobuttersäurenitril) und einem Lösevermittler (z.B. Dichlorethan, Aceton).

Das Reaktionsgemisch wird 5 h bei 60°C und 10 h bei 80°C gerührt. Das entstandene, weiße Polymere wird abgesaugt, mit dest. Wasser gewaschen und getrocknet.

Das entstehende Polymere wird in 10 Gew.-% wässrige Natronlauge eingetragen und 5 h bei 80°C gerührt. Das entstandene Produkt besitzt ionenaustauschende Eigenschaften.

Die nachfolgenden Beispiele sollen das erfindungsgemäße Verfahren verdeutlichen.

Beispiel 1

N-Methyl-N'-phenyl-ethylenharnstoff(-imidazolidinon)

179 g (0,92 Mol) N-2-Hydroxyethyl-N-methyl-N'-phenylharnstoff, 472 g (4 Mol) Diethylcarbonat und 0,5 g Kaliumcarbonat wurden an einer 1,2 m Füllkörperkolonne während 2 Stunden auf 120 bis 135°C Innentemperatur erhitzt, bis über Kopf 70 g Ethanol abdestilliert waren, wobei gleichzeitig $CO_2$ entstand. Nach Abdestillieren des überschüssigen Diethylcarbonats unter 30 mbar erhielt man 180 g Rückstand. Dieser wurde erst unter 6 bis 10 mbar/138-180°C, dann unter 0.01 mbar/125-145°C destilliert. Durch Umkristallisation des Destillats aus Ethanol wurden 97 g kristallines Produkt erhalten, Fp. 109-110°C. Die Ausbeute betrug 60 %, bezogen auf eingesetzten Harnstoff.

Beispiel 2

N-Phenyl-N'-vinyl-ethylenharnstoff

357 g (3 Mol) Phenylisocyanat wurden bei 18 bis 20°C innerhalb von 1 Stunde mit 315 g (3 Mol) Diethanolamin in 1000 g Diethylcarbonat umgesetzt. Nach 1 Stunde wurden weitere 1832 g Diethylcarbonat (insgesamt 24 Mol) zugegeben und nach Zusatz von 1,5 g Kaliumcarbonat wurde an einer 1,2 m Füllkörperkolonne während 6 Stunden auf 120 bis 130°C Innentemperatur erhitzt. Über Kopf wurden 365 g Ethanol abgetrennt, wobei gegen Ende der Umesterung das Ethanol durch stufenweise Verringerung

des Drucks bis auf 300 mbar entfernt wurde. Nach Abdestillieren des überschüssigen Diethylcarbonats unter 30 mbar erhielt man 906 g Rückstand. Dieser wurde unter Eintropfen in einen vorgeheizten Kolben, in den 1 g Kaliumcarbonat vorgelegt wurde, unter Rühren im ölpumpenvakuum bei einer Innentemperatur von 195 bis 230°C gespalten. Es gingen 288 g (51 % Ausbeute, bezogen auf eingesetztes Isocyanat) des gewünschten Produktes über, Fp. 137-140°C (aus Ethanol).

**Beispiel 3**

N-(3,4-Dichlorphenyl)-N'-vinylethylenharnstoff

188 g (1 Mol) 3,4-Dichlorphenylisocyanat wurden bei 18 bis 20°C innerhalb von 1 Stunde mit 105 g (1 Mol) Diethanolamin in 300 g Diethylcarbonat umgesetzt. Nach 1 Stunde wurden weitere 644 g Diethylcarbonat (insgesamt 8 Mol) zugegeben und nach Zusatz von 0,8 g Kaliumcarbonat wurde an einer 1,2 m Füllkörperkolonne während 2,5 Stunden auf 120 bis 135°C Innentemperatur erhitzt. Über Kopf wurden 110 g Ethanol abgetrennt, wobei gegen Ende der Umesterung das Ethanol durch stufenweise Verringerung des Drucks bis auf 300 mbar entfernt wurde. Nach Abdestillieren des überschüssigen Diethylcarbonats unter 30 mbar erhielt man 350 g Rückstand. Dieser wurde, wie in Beispiel 2 beschrieben, gespalten. Dabei gingen 108 g (42 % Ausbeute, bezogen auf eingesetztes Isocyanat) des gewünschten Produktes über. $Kp._{0.02}$ 170-175°C, Fp. 134-136°C (aus Ethanol).

Beispiel 4

N-(3,4-Dichlorphenyl)-N'-(2-ethylcarbonat-ethyl)-ethylenharnstoff

188 g (1 Mol) 3,4-Dichlorphenylisocyanat wurden mit 105 g (1 Mol) Diethanolamin in Diethylcarbonat umgesetzt und mit Diethylcarbonat umgeestert, wie in Beispiel 3 beschrieben. Zur Entfernung des Katalysators wurde das überschüssige Diethylcarbonat nicht entfernt, sondern die Lösung über einen Ionenaustauscher auf der Basis eines sulfonierten Styrol-Divinylbenzol-Copolymerisates gegeben, zum Nachspülen wurde Methylenchlorid verwendet. Das Eluat wurde im Vakuum vollständig eingeengt. Durch Kristallisation aus Ethanol wurden 208 g (60 % Ausbeute, bezogen auf eingesetztes Isocyanat) der Titelverbindung erhalten, Fp. 90-92°C.

Patentansprüche

1) Verfahren zur Herstellung von substituierten Ethylenharnstoffen der Formel

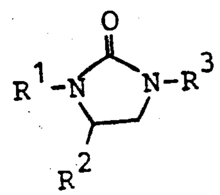

,

worin

$R^1$ für einen gegebenenfalls durch Chlor, Brom, eine Nitro-, Trifluormethyl-, Alkoxygruppe mit 1 bis 4 Kohlenstoffatomen oder eine Carbalkoxygruppe mit 1 bis 4 Kohlenstoffatomen ein- oder mehrfach substituierten Phenylrest steht,

$R^2$ Wasserstoff oder Methyl bedeutet und

$R^3$ für einen gegebenenfalls durch Chlor, Brom, eine Nitro-, Trifluormethyl-, Alkoxygruppe mit 1 bis 4 Kohlenstoffatomen oder eine Carbalkoxygruppe mit 1 bis 4 Kohlenstoffatomen ein- oder mehrfach substituierten Phenylrest, einen Alkylrest mit 1 bis 4 Kohlenstoffatomen, für $-CH_2CHR^2O\overset{O}{\overset{\|}{C}}OR^4$ mit $R^4$ für $C_1$-$C_4$-Alkyl oder für $-CH{=}CHR^2$ und mit der für $R^2$ genannten Bedeutung steht,

dadurch gekennzeichnet, daß man ß-Hydroxyethylharnstoffe der Formel

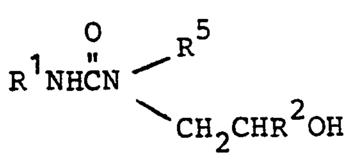

worin

$R^1$ und $R^2$ die oben angegebene Bedeutung haben und

$R^5$ für einen gegebenenfalls durch Chlor, Brom, eine Nitro-, Trifluormethyl-, Alkoxygruppe mit 1 bis 4 Kohlenstoffatomen oder eine Carbalkoxygruppe mit 1 bis 4 Kohlenstoffatomen ein- oder mehrfach substituierten Phenylrest, einen Alkylrest mit 1 bis 4 Kohlenstoffatomen oder für $-CH_2CHR^2OH$ mit der für $R^2$ genannten Bedeutung steht,

mit Kohlensäurealkylestern der Formel

$$R^6O\overset{O}{\overset{\|}{C}}OR^7$$

worin

$R^6$ und $R^7$ gleich oder verschieden sind und für einen Alkylrest mit 1 bis 4 Kohlenstoffatomen stehen,

in Gegenwart von Katalysatoren bei erhöhter Temperatur umsetzt.

2. Verfahren nach Anspruch 1, dadurch gekennzeichnet, daß man die Umsetzung bei Temperaturen von 100 - 270°C durchführt.

3. Verfahren nach Ansprüche 1 und 2, dadurch gekennzeichnet, daß man die Umsetzung bei Temperaturen von 110 - 160°C durchführt.

4. Verfahren nach Ansprüchen 1 bis 3, dadurch gekennzeichnet, daß man als Katalysatoren Verbindungen des Lithiums, Natriums, Kaliums, Rubidiums, Cäsiums, Magnesiums, Calciums, Zinks, Strontiums, Cadmiums, Bariums, Thalliums, Titans, Zinns und/oder des Bleis einsetzt.

5. Verfahren nach Ansprüchen 1 bis 4, dadurch gekennzeichnet, daß man als Katalysatoren die Carbonate, Carboxylate, die Alkoholate und/oder Hydroxide des Natriums und/oder des Kaliums einsetzt.

6. Verfahren nach Ansprüchen 1 bis 5, dadurch gekennzeichnet, daß man die Katalysatoren in Mengen von etwa 0,001 bis 5 Gew.-%, bezogen auf die Menge an ß-Hydroxyethylharnstoff einsetzt.

7. Verfahren nach Ansprüchen 1 bis 6, dadurch gekennzeichnet, daß man die Katalysatoren in Mengen von 0,01 bis 1 Gew.-%, bezogen auf die Menge an ß-Hydroxyethylharnstoff, einsetzt.

8. Neue N-vinylethylenharnstoffe der Formel

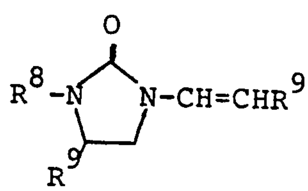 ,

worin

$R^8$ für einen gegebenenfalls durch Chlor, Brom, eine Nitro-, Trifluormethyl-, Alkoxygruppe mit 1 bis 4 Kohlenstoffatomen oder Carbalkoxygruppe mit 1 bis 4 Kohlenstoffatomen ein- oder mehrfach substituierten Phenylrest steht und

$R^9$ Wasserstoff oder Methyl bedeutet.

9. Verwendung der neuen N-Vinylethylenharnstoffe des Anspruchs 8 zur Herstellung von Ionenaustauschern.

Europäisches Patentamt

# EUROPÄISCHER RECHERCHENBERICHT

0131801
Nummer der Anmeldung

EP 84107382.8

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | AT - B - 364 828 (C.H. BOEHRINGER & SOHN)<br>* Anspruch 1 *<br>-- | 1,8 | C 07 D 233/32 |
| A,D | DE - A - 2 035 364 (I.R. GEIGY AG.)<br>* Anspruch *<br>-- | 1,8 | |
| A,D | DE - A - 2 230 076 (CIBA-GEIGY AG)<br>* Ansprüche 1 und 5 *<br>---- | 1,8 | |
| | | | RECHERCHIERTE SACHGEBIETE (Int. Cl.4) |
| | | | C 07 D 233/00 |

Der vorliegende Recherchenbericht wurde für alle Patentansprüche erstellt.

| Recherchenort | Abschlußdatum der Recherche | Prüfer |
|---|---|---|
| WIEN | 05-10-1984 | BRUS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument